# EUROPEAN PATENT SPECIFICATION

(11) **EP 0 765 138 B1**
(45) Date of publication and mention of the grant of the patent: **02.09.1998**
(21) Application number: 95922438.7
(22) Date of filing: 16.06.1995
(51) Int. Cl.: A61B 17/22

(54) **AN APPARATUS FOR FRAGMENTATION OF A LUNG EMBOLUS**
EIN GERÄT ZUR AUFLÖSUNG EINER LUNGENEMBOLIE
APPAREIL POUR DESINTEGRER UN EMBOLUS PULMONAIRE

(30) Priority: 17.06.1994 DE 9409863 U
(43) Date of publication of application: 02.04.1997
(73) Proprietor: WILLIAM COOK EUROPE A/S, 4632 Bjaeverskov (DK)
(72) Inventor: SCHMITZ-RODE, Thomas, D-52070 Aachen (DE); GÜNTHER, Rudolf, W., D-52074 Aachen (DE)
(74) Representative: Indahl, Peter Jensen
(86) International application number: PCT/DK95/00248
(87) International publication number: WO 95/35066

(56) References cited:
- EP-A- 0 123 175
- WO-A-92/03098
- WO-A-93/13820
- DE-A- 3 309 052
- DE-C- 688 834
- US-A- 3 119 392
- US-A- 5 201 741

## Description

The present invention relates to an apparatus for fragmentation of a lung embolus, including a catheter, a guide wire and a flexible bending. Such an apparatus is known from EP-A 549 458.

In the industrial countries the third most frequent cause of death is lung emboli. Ten per cent of the patients with acute lung emboli die from heart failure. Dissolution by medicine of the embolus (thrombolysis) takes hours. The patient often dies before a hemodynamic improvement occurs. Operational removal of the embolus in accordance with the so-called Tendelenburg operation entails a comparatively high rate of mortality.

With a combined treatment comprising mechanical destruction of lung emboli and a subseqent selective thrombolysis of the resulting fragments it has in some cases already been possible to obtain promising treatment results. In this case the fragmentation of an embolus took place by manipulation with diagnostic standard-pulmonalis-catheters. Such a catheter is advanced by means of a guide wire past an embolus towards the periphery. When the catheter after the withdrawal of the wire has taken up its pointed configuration, for instance in form of a so-called pig tail, the embolus is cut into pieces by the withdrawal of the catheter. This process admittedly has to be repeated several times, in order to cause a relatively rough fragmentation of the embolus. By this manouevre a lowering of the pulmonal arterial pressure could be attained, and consequently the occurence of a threatening cardiac shock in the patient with a subsequent death risk could be avoided.

This described fragmentation of pulmonal emboli used in a few cases is not just fairly complicated and protracted, but also very rough, as the standard catheters introduced in that connection do not meet the requirements made to an apparatus for fragmentation of a lung embolus.

However, for the treatment of lung emboli special apparatuses are already known. One of these known from EP-A 549 458 is provided with a rotating basket which can be unfolded by centrifugal force for fragmentation of the embolic material. However, a contact between the unshielded basket treads and the wall of the artery can hardly be avoided and may lead to damage of the artery. In an apparatus known from WO 92/03098 a rotor (impeller) is present in the centre of a self-expanding, stationary protective basket.

In both the apparatuses described above the rotational body is driven through a flexible shaft positioned in the interior of the catheter by an external drive with 100,000 to 150,000 rpm. Thrombotic material in the lung flow path is caught by the created whirl and mechanically fragmented. The high rotational speed, however, means a high load on the material and makes these apparatuses prone to defects. Due to the complexity of both apparatuses, the risk of an erroneous handling by a not optimally trained operator is considerable.

The object of the present invention is to provide an apparatus for fragmentation of a lung embolus, which apparatus has a simple construction and is easy to handle and which simultaneously allows an effective and untraumatic fragmentation treatment of an embolus.

To meet this object the apparatus according to the invention is characterized in that the catheter is torsionally stiff or stable and has a distal end segment preset in the shape of a bending, that through the guide wire the distal end segment is controlable to a straightened configuration, and that when the distal end segment occupies its preset bent configuration an opening on the outer side of said bending is in line with the central axis of the part of the catheter not comprising the bending, which opening has a cross-section being at least slightly larger than the cross-section of the guide wire.

The torsionally stiff or stable catheter is simple and may quickly by means of the guide wire be placed in the Arteria pulmonalis. The guide wire first extends over the whole length of the catheter and does not allow the distal catheter end segment to take up a curved form until the guide wire is withdrawn from the area of the catheter end segment. By a renewed advancing of the guide wire, its distal end segment comes out of the opening which is in line with the centre axis of the catheter in the curved area, and the guide wire thus serves as rotational axis for the torsionally stable catheter. After anchoring of the guide wire further away in the pulmonal artery system the catheter is through its proximal end made to rotate by hand or by means of a mechanical drive. By rotation of the bending of the distal catheter end segment within the embolic blockage the embolic material can be fragmented. The fragmentation may also in addition to rotation take place by moving the catheter forwards and backwards over the stationary guide wire used as a guide member. Thereby the thrombic material is cut into pieces by the bending of the distal catheter end segment passing in quick forwards and backwards movements. On account of the uncomplicated design of the catheter tip and if the usual angiographic precaution measures are taken, no damages to the artery walls are to be expected.

When the catheter end segment occupies its bent configuration, the central axis of the proximal part of the catheter extends out through the opening so that the distal tip of guide wire can catch the opening during advancement of the guide wire. Normally the guide wire tip is rounded or slightly pointed in order to facilitate catching of the opening. With a wire tip shaped in this manner the opening need only be slightly larger than the cross-section of the guide wire extending through the opening, because the guide wire will make the rim of the opening adapt to its own cross-section, once the opening has been caught. It is also possible to make the opening with a suitable overmeassure so that whole or the major portion of the inner lumen of the catheter is exposed by the opening when the distal end segment occupies its bent configuration.

According to a preferred embodiment of the apparatus according to the invention a free end of the bending points towards the central axis of the catheter, whereby the bending is made into an oval loop.

The small diameter of the look is preferably 7 to 10 mm, and the big diameter of the loop is preferably two to three times as big as the small diameter.

In order to be able to use the apparatus according to the invention also for diagnosing a lung embolus, openings are according to a further embodiment of the invention arranged in the distal end segment of the catheter, the cross section of said openings being smaller than the cross section of the guide wire.

The lateral openings known per se in catheters prevent also by their preferred orientation parallel to the axis of the guide wire that the guide wire unintentionally penetrates through one of these openings and impedes a proper handling and positioning of the catheter.

Through the lateral openings a thrombolyticum may also be injected during the fragmentation process or in connection therewith, which thrombolyticum supports the dissolution of the thrombus medicinally. When the guide wire is positioned a so-called Y - rotation adaptor is connected at the proximal catheter end, handle end or motor end. The adabter does not impede the rotation of the cather, it seals the guide wire with respect to the proximal adaptor end and it allows injection of the thrombolyticum through a lateral inlet.

Finally, according to another embodiment of the invention a thread, one end of which protrudes from the proximal end of the catheter, is passed through the catheter, leaves at the distal end of the catheter, is introduced again in the catheter through an opening and protrudes likewise with its other end from the proximal end of the catheter.

By this embodiment the loop formed from the bent distal end segment of the catheter may be stabilized by means of a tightening of the two proximal wire ends, which improves the cutting effect of the bent segment on an embolus.

In the drawing examples of embodiments of the apparatus according to the invention is shown schematically and will be explained in further detail in the following. In the drawing
- Fig. 1: shows the distal end segment of the apparatus
A in unbent condition of the catheter with a guide wire protruding from its end
B the catheter according to A, but with withdrawn guide wire
C the catheter according to B, but after advancing of the guide wire,
- Fig. 2: shows the proximal end of the apparatus
A with a handle threaded onto the catheter
B with an electromotor connected with the catheter,
- Fig. 3: shows the apparatus according to Figs. 1 A - C with a thread running through the catheter lumen
A when the catheter is straightened by the guide wire
B after withdrawal and renewed advancing of the guide wire and tightening of the thread, and
- Fig. 4: shows angled catheter tips for facilitating examination of the right ventricle of the heart and the pulmonal artery.

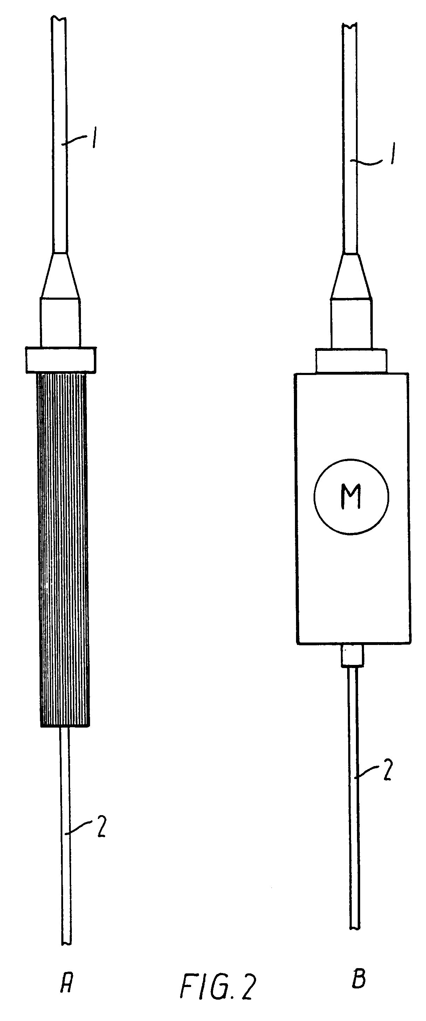

The apparatus consists substantially of a torsionally stiff or stable catheter 1 and a guide wire 2, which forms the rotational axis of the catheter 1, which at its proximal end is provided with a handle or a motor (cf. Fig. 2). Torsionally stiff catheters are well known in the prior art and may e. g. be manufactured by embedding a wire braiding in the catheter wall.

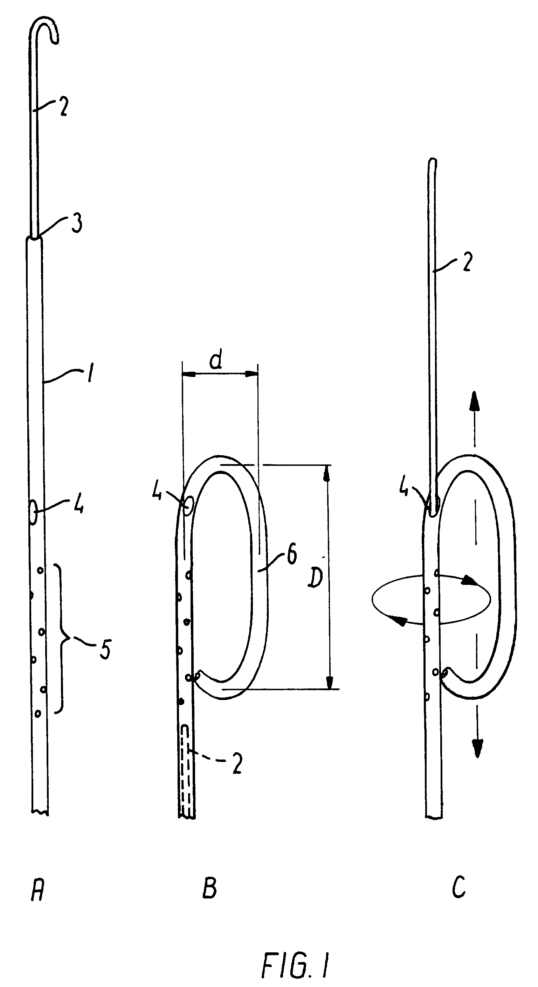

At the introduction in the artery system and during advancing to the right heart atrium the catheter 1 is positioned in the straightened condition on the guide wire 2, shown in Fig. 1. The guide wire protrudes with a portion from the distal end 3 of the catheter 1. When the guide wire is withdrawn, the distal end segment of the catheter 1 enters by elastic restoring forces into the loop configuration 6 shown in Fig. 1 B. This configuration is preferably oval, the small diameter d being from 7 to 10 mm and the big diameter D two or three times as big as d. In this configuraton the right part of the heart and the pulmonal artery can be examined without problems. After positioning of the catheter loop 6 in the lung flow path an angiography may be carried out. Contrast agent comes out of lateral openings 5 for contrast agent and causes a regular contrasting of the artery lumen. After location of the embolus the guide wire 2 is pushed through the catheter 1 and leaves the interior of the catheter through an opening 4 positioned in line with the centre axis of the catheter 1, as shown i Fig. 1 C. The catheter loop 6 hereby retains its shape. The guide wire 2 is in order to stabilize the positioning of the catheter 1 pushed past the embolus and towards the periphery, while the loop is placed in the area of the embolus. By means of the handle or the electromotor at the proximal end the catheter 1 with the loop 6 is made to rotate. The guide wire 2 serves in this connection as a stationary rotational axis. The embolus is fragmented by means of the loop 6, as due to the torsional stability of the catheter the movement of rotation exerted thereon is transferred to the loop 6. The opening 4 is dimensioned so that the guidewire can pass through the opening when the distal end segment is loopshaped. The opening 4 only needs to be slightly larger than the cross-section of the guidewire. When the distal end segment is in the straightened shape, the opening 4 preferably has an oval shape with its major axis extending in parallel with the longitudinal axis of the catheter.

By using a guide wire 2 with a movable core and J-tip with narrow bending, the loop 6 of the catheter 1 may once again become straightened by renewed advancing of the guide wire 2 with resilient distal end segment of the guide wire 2, as the guide wire 2 with resilient narrow bending during advancing follows the course of the central axis of the catheter also in the area of the cathether segment with the opening 4, so that the guide wire tip passes the opening and is moved into the bent distal end segment of the catheter. Then the movable core may be advanced and the distal end segment straightened. Such a handling may be necessitated for a repositioning or for a further peripheral examination with the catheter. By alternatively advancing the guide wire 2 with stiff distal end, it comes out through the opening 4 of the catheter.

During or after fragmentation thrombolyticum may be introduced directly in the emboulus through the openings 5 or may be injected in the flow area containing the fragments.

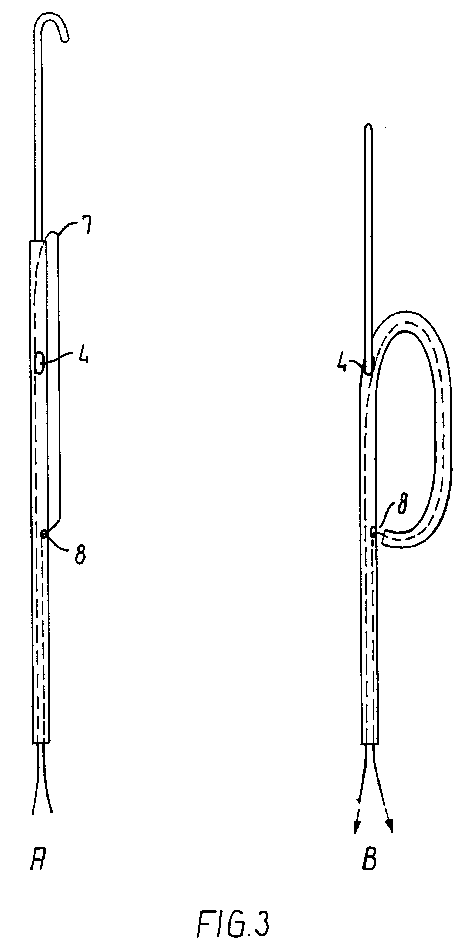

According to Fig. 3 a thin, non-stretch thread 7 is passed through the interior of the catheter and comes out at the distal end of the catheter 1 and returns to the interior of the catheter through an opening 8 and runs back to the proximal end of the catheter 1. If the thread 7 is tightened, the loop 6 according to Fig. 3 B is stabilized by anchoring of the catheter distal end opposite the opening 8.

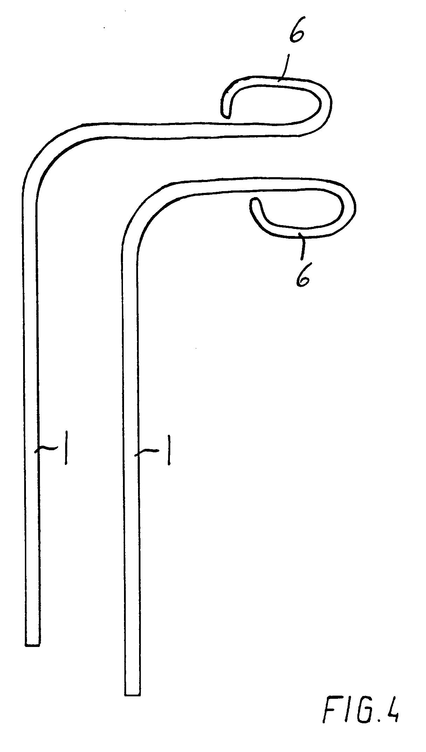

An angling of a distal end segment of the catheter 1, as shown in Fig. 4, facilitates the examination of the right heart ventricles and the central pulmonal arteries.

The braiding in the catheter wall may preferably extend to a position just proximal of the opening 4, leaving the bendable distal segment of the catheter free of such braiding. The distal end segment is by well known techniques set in the desired loop shape, and the elastic properties of the segment allows it to be straightened by the guide wire.

It is alternatively possible to let the braiding partially extend into the bendable distal end segment in the area at the inside of the bending in order to strengthen the catheter at the transition from the proximal to the distal side of the opening 4. In this case the braiding extending into the bendable distal end segment may also be preset in the shape of the loop 6.

## Claims

1. An apparatus for fragmentation of a lung embolus, including a catheter (1), a guide wire (2) and a flexible bending, **characterized** in that the catheter (1) is torsionally stiff or stable and has a distal end segment preset in the shape of a bending, that through the guide wire (2) the distal end segment is controlable to a straightened configuration, and that when the distal end segment occupies its preset bent configuration an opening (4) on the outer side of said bending is in line with the central axis of the part of the catheter not comprising the bending, which opening has a cross-section being at least slightly larger than the cross-section of the guide wire (2).

2. An apparatus according to claim 1**, characterized** in that a free end of the bending (6) points towards the central axis of the catheter (1) and that the bending is made into an oval loop (6).

3. An apparatus according to claim 2**, characterized** in that the small diameter (d) of the loop (6) is approximately 7 to 10 mm and the big diameter (D) of the loop (6) is two or three times as big as the small diameter (d).

4. An apparatus according to any of claims 1 to 3, **characterized** in that openings (5) are arranged in the distal end segment of the catheter (1), the cross section of said openings being smaller than the cross section of the guide wire (2).

5. An apparatus according to anyone of claims 1 to 4, **characterized** in that a thread (7), one end of which protrudes from the proximal end of the catheter, is passed through the catheter (1), leaves at the distal end of the catheter (1), is introduced again in the catheter (1) through an opening (8) and protrudes likewise with its other end from the proximal end of the catheter (1).

## Patentansprüche

1. Vorrichtung zur Fragmentation eines Lungenembolus, die einen Katheter (1), einen Führungsdraht (2) und eine elastische Krümmung einschließt, dadurch gekennzeichnet, daß der Katheter (1) torsionssteif oder -stabil ist und ein distales Endstück aufweist, dessen Krümmungsform vorgegeben ist, daß durch den Führungsdraht (2) das distale Endstück in eine gestreckte Form bringbar ist und daß, wenn das distale Endstück seine vorgegebene gekrümmte Form einnimmt, eine Öffnung (4) auf der Außenseite der Krümmung mit der Mittelachse des die Krümmung nicht aufweisenden Katheterteils ausgerichtet ist, wobei die Öffnung einen Querschnitt hat, die zumindest geringfügig größer ist als der Querschnitt des Führungsdrahtes (2).

2. Vorrichtung nach Anspruch 1, dadurch gekennzeichnet, daß ein freies Ende der Krümmung (6) zur Mittelachse des Katheters (1) weist und die Krümmung zu einer ovalen Schlinge (6) vervollständigt ist.

3. Vorrichtung nach Anspruch 2, dadurch gekennzeichnet, daß der kleine Durchmesser (d) der Schlinge (6) etwa 7 bis 10 mm und der große Durchmesser (D) der Schlinge (6) das 2- bis 3fache des kleinen Durchmessers (d) beträgt.

4. Vorrichtung nach einem der Ansprüche 1 bis 3, dadurch gekennzeichnet, daß im distalen Endstück des Katheters (1) Öffnungen (5) vorgesehen sind, deren jeweiliger Querschnitt kleiner ist als der Querschnitt des Führungsdrahtes (2).

5. Vorrichtung nach einem der Ansprüche 1 bis 4 dadurch gekennzeichnet, daß ein mit seinem einen Ende über das proximale Ende des Katheters (1) vorstehender Faden (7) durch den Katheter (1) hindurchgeführt ist, am distalen Ende des Katheters (1) austritt, durch eine Öffnung (8) erneut in den Katheter (1) eingeführt ist und mit seinem anderen Ende gleichfalls über das proximale Ende des Katheters (1) vorsteht.

## Revendications

1. Appareil pour la fragmentation d'un embole pulmonaire comprenant un cathéter (1), un fil de guidage (2) et un cintrage souple, caractérisé en ce que le cathéter (1) est rigide en torsion et/ou stable et présente un segment d'extrémité distale préétabli sous la forme d'un cintrage, en ce que par le fil de guidage (2), le segment d'extrémité distale peut être commandé pour prendre une configuration redressée et en ce que lorsque le segment d'extrémité distale occupe sa configuration cintrée préétablie, une ouverture (4) sur le côté extérieur du cintrage est en alignement sur l'axe géométrique central de la partie du cathéter ne comprenant pas le cintrage, laquelle ouverture a une section transversale au moins légèrement plus grande que la section transversale du fil de guidage (2).

2. Appareil selon la revendication 1, caractérisé en ce qu'une extrémité libre du cintrage (6) est dirigée vers l'axe géométrique central du cathéter (1) et en ce que le cintrage forme une boucle ovale (6).

3. Appareil selon la revendication 2, caractérisé en ce que le petit diamètre (d) de la boucle (6) est d'environ 7 à 10 mm et en ce que le grand diamètre (D) de la boucle (6) est deux ou trois fois aussi grand que le petit diamètre (d).

4. Appareil selon l'une quelconque des revendications 1 à 3, caractérisé en ce que des ouvertures (5) sont disposées dans le segment d'extrémité distale du cathéter (1), la section transversale des ouvertures étant inférieure à la section transversale du fil de guidage (2).

5. Appareil selon l'une quelconque des revendications 1 à 4, caractérisé en ce qu'un fil (7) dont une extrémité fait saillie à partir de l'extrémité proximale du cathéter, est passé à travers le cathéter (1), sort au niveau de l'extrémité distale du cathéter (1), est introduit à nouveau dans le cathéter (1) par une ouverture (8) et fait saillie de la même manière par son autre extrémité par l'extrémité distale du cathéter (1).
